(19) Europäisches Patentamt

European Patent Office

Office européen des brevets

(11) **EP 1 033 138 B1**

(12) **EUROPEAN PATENT SPECIFICATION**

(45) Date of publication and mention
of the grant of the patent:
**21.06.2006 Bulletin 2006/25**

(51) Int Cl.:
*A61L 2/10* (2006.01)    *A61L 12/06* (2006.01)
*B65B 55/08* (2006.01)    *G02C 7/04* (2006.01)

(21) Application number: **00301642.5**

(22) Date of filing: **01.03.2000**

(54) **Method of sterilization**

Sterilisationsmethode

Méthode de stérilisation

(84) Designated Contracting States:
**DE FR GB IE IT**

(30) Priority: **01.03.1999 US 259758**

(43) Date of publication of application:
**06.09.2000 Bulletin 2000/36**

(73) Proprietor: **Johnson & Johnson Vision Care, Inc.
Jacksonville, FL 32256 (US)**

(72) Inventors:
• **Brown-Skrobot, Susan K.
Jacksonville,
Florida 32256 (US)**
• **Beaton, Stephen R.
Jacksonville,
Florida 32250 (US)**
• **Bussey, Eric P.
Middleburg,
Florida 32068 (US)**
• **Ebel, James A.
Jacksonville,
Florida 32256 (US)**
• **Hill, Gregory A.
Atlantic Beach,
Florida 32223 (US)**

• **Peck, James
Jacksonville,
Florida 32224 (US)**
• **Heaton, John C.
Atlantic Beach,
Florida 32233 (US)**
• **Cristol, David A.
Jacksonville,
Florida 32256 (US)**
• **Newton, Timothy P.
Jacksonville,
Florida 32256 (US)**
• **Enns, John B.
Jacsonville,
Florida 32257 (US)**

(74) Representative: **Mercer, Christopher Paul
Carpmaels & Ransford
43, Bloomsbury Square
London WC1A 2RA (GB)**

(56) References cited:
**EP-A- 0 479 723          WO-A-97/43915
US-A- 5 768 853**

**Description**

FIELD OF THE INVENTION

This invention relates broadly to sterilization of contact lenses. More specifically, this invention relates to a novel process for the sterilization of contact lenses using ultraviolet radiation.

DESCRIPTION OF THE RELATED ART

Medical device sterilization processes, and in particular commercial contact lens manufacturing sterilization processes, typically involve some form of temperature and/or pressure-based sterilization techniques. For example, a hydrophilic contact lens is typically first formed by injecting a monomer mixture into a mold. The monomer mixture is then polymerized (i.e. the lenses are cured). After other optional processing steps, such as quality inspections, the lens is placed into a container with a solution and the container is sealed. The packaged lens is sterilized by placing the container into an autoclave at an elevated temperature and pressure for an extended period of time, usually at least 15 minutes, typically 30 minutes. Although this commercial process produces thoroughly sterilized contact lenses, the batch-wise autoclave sterilization step is time consuming and costly.

European Patent Application No. 0 222 309 A1 discloses a process using ozone in which packaging material is disinfected in a manufacturing setting. The process involves feeding an oxygen stream into an ozonating chamber, generating ozone from oxygen in the ozonating chamber, placing packaging containers in a sanitizing chamber, feeding the ozone into the sanitizing chamber, and purging the ozone from the sanitizing chamber with sterile air. The process requires that the ozone contact the packaging material for a predetermined time, followed by the sterile air purge step. The process is offered as an alternative to heat-steam sterilization, sterilization by application of electromagnetic radiation, or chemical agent sterilization.

U.S. Patent No. 5,618,492 discloses a process for producing a sterile contact lens in a sealed container during a continuous production process wherein the contact lens is immersed in an ozone-containing solution within a container during a continuous lens packaging process, and the lens and container are subsequently subjected to ultraviolet radiation primarily to degrade the ozone. This process sterilizes the contact lens and the container.

Non-ionizing radiation such as ultraviolet (UV) light is known to damage the DNA of exposed cells. The UV light causes bonds to form thymine dimers which inhibit replication of DNA during cell reproduction. UV light is used for disinfection in hospital rooms, nurseries, operating rooms and cafeterias. UV light is also used to sterilize vaccines, serum, toxins, municipal waste, and drinking waters. The major weakness of the efficacy of UV light as a sterilizer is that for most materials the radiation is not very penetrating, so the microorganisms to be killed must be directly exposed to the radiation.

A number of patents teach the application of UV light to disinfect and/or inactivate microorganisms to either reduce populations of microorganisms or to eliminate them.

US Patent 5,768,853 and WO96/09775 describe the use of a UV light producing apparatus which deactivates microorganisms in food.

US Patent 4,464,336 suggests a method of sterilization by using a flash discharge ultraviolet lamp. The patent teaches that by applying short duration high intensity UV light that microorganisms will be destroyed; however, the conditions for sterilization are not disclosed, nor its application for medical devices

US Patent 5,786,598 and WO 97/43915 disclose the broad concept that a flash lamp system might be used for deactivating microorganisms in containers including a polyolefin container with a foil backing that contains a contact lens and a preservative fluid. Preservation is the use of physical and/or chemical means to kill or prevent the growth of those microorganisms which, by their growth and/or activities, may cause bio-deterioration of a given material or product. P. Singleton and D. Sainsbury, 1988. Dictionary of Microbiology and Molecular Biology, John Wiley & Sons, New York, NY, pp. 702-703. Although the patent discloses the idea of using a flash lamp system to sterilize contact lenses in a preserved solution in a container, there are no conditions defined to accomplish sterility, nor examples which show that sterility can be accomplished.

U.S. Patents 5,034,235 and 4,871,559 disclose the use of intermittent pulses of very intense, very short duration pulses of light to inactivate microorganisms on the surface of food products, and suggests that the method can be used for packages, medical devices, and food products in packages.

There still remains a need for a time-efficient, continuous, effective sterilization process for pharmaceutical, medical, and cosmetic products which can be used in an inline mode in the manufacture of these products.

SUMMARY OF THE INVENTION

This invention provides a process according to claim 1.

DESCRIPTION OF THE FIGURES

The invention will be described with reference to the following figures:

Figures 1 and 2 illustrate one embodiment of the system used in the process of this invention;
Figure 3 shows the sensitivity of Aspergillus niger (ATCC 16404) to radiation of different spectral ranges;
Figure 4 shows the sensitivity of Bacillus stearothermophilus (ATCC 7953) to radiation of different spectral ranges;
Figure 5 shows the sensitivity of Pseudomonas aeruginosa (ATCC 9027) to radiation of different spectral ranges; and
Figure 6 shows a graph used to determine the $D_{value}$ of Bacillus stearothermophilus (ATCC 7953).

DESCRIPTION OF THE INVENTION

The term "sterile" or "sterilization" as used herein means the condition of an object, or an environment, which is free of all living cells, all viable spores (and other resistant and disseminative forms), and all viruses and subviral agents capable of replication. Sterility is assured by a minimum sterility assurance level of $10^{-3}$, preferably $10^{-6}$, more preferably $10^{-9}$, and most preferably $10^{-12}$ when the container is inoculated with $10^{6}$ microorganisms. The minimum sterility assurance level is dependent on the type of medical device. For example, for sterilization of a single-use contact lens, the USFDA requires a minimum sterility assurance level of $10^{-6}$ in the number of microorganisms per container. A sterility assurance level of $10^{-6}$ is the probability of having 1 non-sterile package out of one million packages.

The "$D_{value}$" is the amount of energy required to kill 90 percent of the organisms present. For steam sterilization, the $D_{value}$ is the time required at a given temperature to reduce the number of viable cells or spores of a given microorganism to 10 percent of the initial number, according to P. Singleton and D. Sainsbury in Dictionary of Microbiology and Molecular Biology, 1988, John Wiley & Sons, New York, NY, pg. 256. According to ANSI standards for gamma radiation, the $D_{value}$ is the radiation dose required to kill 90 percent of the organisms of a homogeneous microbial population, which is defined by assuming that the death of microbes follows first order kinetics. In the case of non-ionizing radiation, the $D_{value}$ will be the non-ionizing radiation dose required to kill 90 percent of the organisms of a homogeneous microbial population, which is defined by assuming that the death of microbes follows first order kinetics. The $D_{value}$ is calculated using the Stumbo-Murphy-Cochran Equation: $D_{value}=U/(Log\ N_0-Log\ NJ$, where No is the initial number of microorganisms in each replicate unit, $N_u$ is ln(n/r), where n is the total number of replicate units exposed to the sterilizing dose U, and r is the number of units exposed to dose U that test negative for growth. Using the $D_{value}$, the sterilizing dose can be calculated. For sterilization of a medical device such as a single-use contact lens, the requirement is for a sterility assurance level of $10^{-6}$. The total dose, therefore equals $D_{value}$ (log $N_o$-log $N_u$).

The term "ultraviolet radiation" or "UV radiation" means radiation having a wavelength or wavelengths between from 200 to 400 nm. If a range is specified following the term "ultraviolet radiation" or "UV radiation", a narrower range of radiation is meant within the 200 to 400 nm range. Further, the range specified, unless otherwise stated, means radiation having a wavelength or wavelengths within the range.

The term "broad spectrum of radiation" means radiation having at least a majority of the wavelengths between 200 to 1100 nm wherein at least a portion of the radiation is UV radiation.

To decrease the complexity and energy demands of this method of sterilization, it would follow that it would be preferred that the contact lens is at least partially transmissive to ultraviolet radiation, preferably it is transmissive to at least 10% of the ultraviolet radiation, more preferably it is transmissive to at least 25% of the ultraviolet radiation, and most preferably the it is transmissive to at least 50% of the ultraviolet radiation. The medical device treated by the method of this invention is a contact lens. The contact lens is in a hermetically sealed contact lens container, and it is preferred that the contact lens container holds a liquid in which the contact lens is immersed. Even though the method would be simplified by using a UV transmissive medical device, the presently preferred contact lens is a contact lens comprising a UV-blocker which blocks greater than 30%, more preferably greater than 50%, and most preferably greater than 80% of the UV radiation (240-280 nm) impinging upon it.

It is preferred that the process of this invention is incorporated into an in-line continuous contact lens manufacturing and packaging process. In the most preferred embodiment, the contact lens is formed and placed in a contact lens container, solution is added to the container, the container is sealed and the container is subjected to short duration, high intensity pulses of radiation, including ultraviolet radiation (240-280 nm)to produce a sterile packaged contact lens which is ready for distribution and use. In the preferred embodiment, the contact lens comprises a hydrogel material and the contact lens is stored in an aqueous solution in the container. The manufacture and placement of the contact lens in the container can be by any process for making contact lenses including those described in, for example, US Patents 5,435,943; 5,395,558; 5,039,459; 4,889,664; 4,565,348; 4,495,313. Other methods are disclosed in other patents, and are known to a person of ordinary skill in the art.

The preferred method provides that a contact lens mold is formed by injection molding two thermoplastic contact lens mold halves which, when put together, form a cavity in the shape of the contact lens. These thermoplastic contact lens molds are typically used once to form a contact lens. Reusable lens molds made out of more durable materials, for example, glass or metal can also be used. Typically, before the lens molds are put together, the monomer or prepolymer mixture which forms the contact lens polymer is injected into a first mold half and a second mold half is placed onto the first mold half which pushes out any excess monomer or prepolymer mixture. However, a one-piece mold can be used to form the contact lens, or the monomer or prepolymer mixture can be injected between the molds after assembly of the molds. The monomer or prepolymer mixture is then cured to form the contact lens. Curing of the monomer or prepolymer mixture is preferably initiated by using photoinitiation. After curing the monomer or prepolymer mixture, the mold halves are removed, and the contact lens is hydrated, if needed. After hydration, preferably one contact lens is placed in a contact lens container. It is preferred that each contact lens container also contains at least enough aqueous solution to fully wet the contact lens in the container, but the presence of aqueous solution is not required in the process of this invention. The aqueous solution, if present, can be added to the container before or after placing the contact lens in the container. Further, the container can be sealed before or after the sterilization step. Alternatively, the container can be sealed before and after the sterilization step if multiple layers of the container are added in multiple steps. For example, a sealed container can be shrink-wrapped or a label added after the sterilization step; or there can be multiple sterilization steps before and after sealing the container. For example, the container can undergo the sterilization step while empty, and/or when it has liquid in it and/or when it has a contact lens placed in it and is not sealed, and/or after the container is sealed with the contact lens and optional liquid present in the container. In additional embodiments, the individual parts and contents of the container can be individually treated by the method of this invention, and then assembled, and/or treated again. It is presently preferred to hermetically seal the container in which a contact lens is present prior to the process of sterilization, and to perform the step of subjecting the hermetically sealed container having the contact lens therein to UV radiation (240-280 nm) only once. The container can be sealed by any means which provides a hermetically sealed container.

The contact lenses useful in accordance with the present invention may be formed from any materials useful for contact lenses. For example, the lenses may be hydrophilic lenses formed from the polymerization or copolymerization of acrylates or methacrylates, such as 2-hydroxyethyl methacrylate (i.e., HEMA); hydrophobic lenses formed from polysiloxanes; or lenses formed from copolymers displaying a range of hydrophobic and hydrophilic properties. The preferred contact lens material is Etafilcon-A which comprises HEMA, MAA, EGDMA, TGDMA, and Darocur. Useful contact lens materials are described in US Patents 5,484,863; 5,039,459; 4,889,664; 5,0684,058; 5,654,350; 5,648,402; 5,311,223; 5,304,584; 5,256,751; 5,196,458; 4,495,313; and 4,680,336.

Additionally, the process of this invention can be used to sterilize contact lenses which contain ultraviolet radiation blocking agents. Contact lenses containing ultraviolet radiation blocking agents include, Acuvue® and Surevue® made by Johnson & Johnson Vision Products. Blocking agents which can be used in contact lens compositions include Norbloc™ 7966, which is 2-(2'-hydroxy-5-methacryloxyethylphenyl)-2H-benzotriazole; other benzotriazoles, such as, 2-(2'-hydroxy-5'methacrylyloxyethylphenyl)-2H-benzotriazole; 2-{3'-tert-butyl-2'-hydroxy-5'-(3"methacryloyloxypropyl) phenyl}-5-chlorobenzotriazole; and benzophenones, such as, 1,3-bis(4-benzoyl-3-hydroxyphenoxy)-2-propyl)(meth) acrylate; 2-hydroxy-4-((meth)acryloxyethoxy)benzophenone; 4-methacryloxy-2-hydroxybenzophenone, others disclosed in US Patents 5,133,745; 4,528,311; 4,716,234; 4,528,311 and 5,681,871, and others known to a person of ordinary skill in the art. Contact lenses can be made according to the examples and teachings in US Patents 5,133,745; 4,716,234; 4,528,311; 4,304,895 and 5,681,871. Also the process of this invention can be used to sterilize contact lenses which contain tints, including visibility tints, including Reactive Blue #4 (2-anthracenesulfonic acid, 1-amino-4-(3-((4,6-dichloro-s-triazin-2-YL)amino)-4-sulfoanilino)9,10-dihydro-9,10-dioxo-) and other materials disclosed in U.S. Patent 5,292,350. The most preferred lens material is Etafilcon-A further comprising Norbloc™ and Reactive Blue #4.

The aqueous solution, if present in the contact lens container, is preferably buffered to a pH of between about 6.5 to about 7.8 in order to approximate the pH of the fluids in the eye. The solution may be buffered by a wide variety of buffers including phosphates, borates, citrates, and other biocompatible buffers known to a person of ordinary skill in the art. The presently preferred buffer is a borate solution. The amount of solution depends on the size of the container. The solution preferably does not contain any preservatives. Typically, the container has a volume between 0.5 milliliters and 50 milliliters, preferably about 1 milliliter, and there is between 0.1 milliliter to 1 milliliters, preferably about 0.5 milliliters of solution in the container.

Containers which are useful in this invention are any of the known containers which are or can be hermetically sealed as long as the containers are at least partially transmissive to ultraviolet radiation (240-280 nm). The containers can be UV transmissive glass, thermoplastic pouches and bags, cyclic olefin copolymers, injection molded or thermoformed plastic containers, and conventional bowls and lids for contact lenses, as long as enough ultraviolet radiation (240-280 nm) can penetrate the container to sterilize the contents of the container. It is presently preferred that the contact lens container comprises a bowl and a lid. It is preferred that the material or materials of at least the bowl of the container are at least partially transmissive to ultraviolet radiation. Particularly, for the sterilization of a contact lens comprising UV-blocker, it is even more preferred that the bowl and the lid are at least partially transmissive to ultraviolet radiation, preferably in all directions. To accomplish this, it is preferred to replace the conventional foil lid with a thermoplastic lid, which may consist of one or more layers of, for example, Aclar, nylon, polyamide, polyvinylidenefluorides (PVDF), polyvinylchlorides (PVC), Saran (polyvinylidene chloride (PVDC)), Saran coated PVC, polyfluorides, e.g. polytetrafluorethylene, cast polypropylene, acrylics, polyethylenes, polystyrenes, and polychlorofluorides e.g. polychlorotrifluoroethylene, polyesters, and copolymers of these materials and cyclic olefin copolymers. It is presently preferred that the bowl is a polyolefin, and the lid comprises polypropylene. The materials of the bowl and the lidstock should preferably be free of any component that will scatter light. The most preferred method of sealing the container is to heat seal the thermoplastic lid to the thermoplastic bowl. The most preferred containers and materials for the container are described in a simultaneously filed European application by the same applicant, claiming priority from U.S. Serial No. 09/259 795 entitled "Package for Medical Device" (attorney's ref; P024043EP).

Sterilization of the contact lens and preferably the contents of the container is achieved by subjecting the container to short duration, high intensity radiation comprising ultraviolet radiation (240-280 nm), wherein the energy density of said high intensity ultraviolet radiation (240-280 nm) inside said container is sufficient to provide complete inactivation of the respective microorganism. The radiation must comprise UV radiation (240-280 nm), because it has been determined that radiation in the ultraviolet radiation range is most responsible for the inactivation of microorganisms; however, the IR, and VIS areas of the spectrum may or may not contribute to the inactivation depending on the microorganism. Different microorganisms are known to be affected differently by ultraviolet radiation (240-280 nm). Viruses are susceptible to UV radiation, and vegetative bacteria are more susceptible to UV radiation. The spore-forming microorganisms are known to be the most resistant to UV radiation. The reasons for the resistance of the spores to UV radiation is primarily attributed to the composition of the outer coat of the spore and the ability of spores to refract light. Therefore, a range of intensities will affect different types of microorganisms differently. We have determined that one of the most resistant microorganism to this method of sterilization is <u>Bacillus stearothermophilus</u> (ATCC 7953). The $D_{value}$ for <u>Bacillus stearothermophilus</u> (ATCC 7953) is at least 3.9 mJ/cm$^2$ ultraviolet radiation (240-280 nm) to the spores. For a sterility assurance level of 10$^{-3}$, the dose to the microorganisms (where the initial inoculum is 10$^4$ cfu/container) is at least 30 mJ/cm$^2$ of UV radiation (240-280nm). For a sterility assurance level of 10$^{-6}$, the dose to the microorganisms is at least 41 mJ/cm$^2$ of UV radiation (240-280nm). For a sterility assurance level of 10$^{-9}$, the dose to the microorganisms is at least 52 mJ/cm$^2$ of UV radiation (240-280nm). For a sterility assurance level of 10$^{-12}$, the dose to the microorganisms is at least 65 mJ/cm$^2$ of UV radiation (240-280nm). This energy is preferably supplied in a single flash by a flash lamp. For an initial inoculum of 10$^6$, the microorganisms are exposed to at least 45 mJ/cm$^2$ of said UV radiation (240-280nm) to provide a sterility assurance level of 10$^{-6}$. In the preferred embodiment, in which the medical device is a UV-blocker lens in a container having approximately 50% transmissivity for UV radiation (240-280 nm), the energy is provided by two flash lamps each having a reflector and each of which provides approximately 3.5 J/cm$^2$ broad spectrum of which 50 mJ/cm$^2$ is UV radiation (240-280 nm) to the container, and the lamps flash simultaneously, whereby the contact lens, and more preferably the contact lens and the contents of the container are rendered sterile. The container permits approximately 50 percent of the UV radiation impinging on it from each lamp to reach the contents of the container.

The high intensity ultraviolet radiation can be generated and directed to the container by any means including lasers, and lamps, with or without reflectors, lightguides, lightpipes, fiber optics, dioptric or catadioptric optical systems to focus light on the product. It is preferred that at least 10%, more preferred at least 30% and most preferred at least 50% or more of the total energy which reaches all the surfaces of the medical device to be sterilized is ultraviolet radiation (240-280 nm). For a medical device in a container, it is preferred that at least 10%, more preferred at least 30% and most preferred at least 50% or more of the total energy which reaches all the surfaces of the medical device, and the contents of the container is UV radiation (240-280 nm). The high intensity ultraviolet radiation, generated by any means including lasers, and lamps, pulsed or continuous, can be adjusted by the use of filters, choppers, splitters, optics, dichroic mirrors, diffractive mirrors, gratings, bandpass filters, short pass filters, long pass filters, dielectric coatings, multilayer coatings, selective beamsplitters, monochromators, Fabry-Perot Etalon, reflective coatings, binary optics, acoustic modulators, or prismatic devices to focus the radiation on the container, or to limit the radiation which reaches the medical device or the container to the germicidally effective wavelengths, or to eliminate radiation at wavelengths which will detrimentally impact the container polymer or medical device polymer. For many polymers which are commonly used for contact lens containers and contact lenses, which were described earlier, radiation at wavelengths less than 320 nm is absorbed by the polymers and may cause chain scissions within the polymers. To protect the polymers from degradation, the means to adjust the wavelengths of the radiation, which reaches the contact lens container, can be present between the radiation source and the contact lens container, or the container can be constructed to filter out certain regions of the spectrum.

Surprisingly, it was found that the presence of UV-blocker agents in the contact lens polymer provides protection to the contact lens polymer. For example, an Etafilcon-A contact lens without UV blocker can withstand up to approximately 35 mJ/cm$^2$ UV radiation (240-280 nm) and/or approximately 5 J/cm$^2$ broad spectrum radiation delivered to each side of the lens by each of two flash lamps which flash simultaneously in multiple flashes before the equilibrium water content of the contact lens is increased such that it falls out of specification; however, an Etafilcon-A contact lens comprising a UV blocker, such as, Norbloc™ can withstand more than 150 mJ/cm$^2$ UV radiation (240-280 nm), and/or approximately 10 J/cm$^2$ broad spectrum radiation delivered to the contact lens before its mean equilibrium water content is increased such that it falls out of specification.

The sterilization dose of radiation can consist of only ultraviolet radiation having wavelengths of 200 to 400 nm, or between from 240 to 280 nm, or radiation at a single wavelength, or the radiation can consist of a broad spectrum of wavelengths including visible, infrared, or any combination of the above, as long as at least a portion of the spectrum is at 254 nm or between from 250 to 260 nm. In the presently preferred embodiment, each flash lamp produces 3.5 J/cm$^2$ broad radiation of which approximately 11.9% of the total energy is between from 200 to 400 nm, 1.8% is between from 240 to 280 nm, and 0.5% is between from 250 to 260 nm. A more preferred radiation source would be one in which at least 25% of the total energy is between from 240 to 280 nm; more preferably at least 50% and most preferably greater than 90% of the total energy is between from 240 to 280 nm.

Sterilization of the container, which preferably comprises at least one contact lens and aqueous solution is preferably achieved by subjecting the contact lens and container to short duration, high intensity ultraviolet radiation, wherein the energy density of said high intensity ultraviolet radiation in the range of 240-280 nm at all the surfaces of the contact lens is at least 18 mJ/cm$^2$, more preferably at least 30 mJ/cm$^2$, most preferably at least 36 mJ/cm$^2$, whereby the contact lens, and more preferably the contact lens and the contents of the container are rendered sterile.

Note that the monitoring system that was used to measure all the spectroradiometirc energies reported herein is further described in a simultaneously filed European application by the same applicant, claiming priority from U.S. Serial No. 09/259 796 entitled "Sterilization System" (Attorney's ref: P024044EP).

The monitoring system disclosed in Ebel et al, can measure differences in the spectroradiometric output of each flash, even if the total energy level of the flash does not change. That application indicates in an example the importance of measuring the spectroradiometric radiation, because in the preferred embodiment, a measurement of the broad spectrum radiation does not indicate if the amount of UV radiation (240-280 nm) as a portion of the total radiation has dropped below the necessary amount to achieve sterilization.

In the preferred embodiment, the ultraviolet radiation is delivered to the container by a system which pulses or flashes the container with a broad spectrum of radiation including ultraviolet radiation. The pulses or flashes are short and intense, that is, the pulses last less than 5 seconds, more preferably less than 3 seconds, and most preferably less than 1 second. In the preferred system each flash lasts less than 0.5 second, more preferably less than 0.1 second, most preferably about 1 millisecond, and the time between flashes is about 100 to about 200 milliseconds; therefore, the sterilization process can be complete within seconds even if it takes multiple flashes or pulses to deliver the sterilizing dose. The pulses or flashes deliver enough energy to sterilize the medical device and contents of the container. Unlike continuous ultraviolet radiation which delivers a uniform amount of energy over a period of time, each flash or pulse of energy in this system delivers a large amount of energy to surfaces of the contact lens and the container in a short period of time. It is preferred that at least 18 mJ/cm$^2$ (240-280nm) reaches the surface of the contact lens in less than 10 millisecond, more preferably in less than 1 millisecond, most preferably in less than 500 microseconds. More preferably, at least 30 mJ/cm$^2$ reaches the microorganisms in less than 1 millisecond. It has been found that the effectiveness of the system at sterilizing is dependent not only on the total amount of energy that is supplied to the container, but that there is a maximum amount of time (or flashes) in which that energy needs to be delivered to the container. It has been determined that the energy required to render the contact lens sterile is preferably delivered to the surface of the contact lens in at most three flashes, more preferably at most two flashes and most preferably in one flash of at least one radiation source in a pulsed UV radiation system. It is preferred that the flash lamp apparatus or each flash lamp in the preferred embodiment provides at least 20 mJ/cm$^2$, more preferably at least 25 mJ/cm$^2$, and most preferably at least 30 mJ/cm$^2$ UV radiation (240-280 nm) in each flash to the microorganisms. It is also preferred that the flash lamp apparatus or each flash lamp in the preferred embodiment produces at least 40 mJ/cm$^2$, more preferably at least 50 mJ/cm$^2$ and most preferably at least 60 mJ/cm$^2$ UV radiation (240-280 nm) in each flash. For a medical device in a container, the medical device having less than 20 percent transmissivity (240-280 nm), it is preferred that multiple radiation sources flash simultaneously to produce at least 80 mJ/cm$^2$ total UV radiation (240-280 nm) per flash, more preferably at least 100 mJ/cm$^2$ total UV radiation (240-280 nm) per flash, and most preferably at least 120 mJ/cm$^2$ total UV radiation (240-280 nm) per flash which impinges on the container.

Additional benefits of a pulsed ultraviolet radiation system over a continuous ultraviolet radiation system is that it generates less heat, and that the system can be monitored to verify that a suitable spectrum was generated for every flash, using the monitoring system disclosed in the "Sterilization System" application referred to above.

The application of the short, high intensity ultraviolet radiation can be accomplished by using one or more sources of radiation. If one source of radiation is used it is preferably directed at the medical device at the base, preferably the bowl, of the container. If two sources of radiation are used, preferably one is directed at the medical device through the base and the other is directed through at the top, preferably at the lid of the container. As long as the contents of the container receives a sterilizing dose of radiation, and each single cell of microorganism is exposed to sufficient energy to inactivate it, the configuration of the sources of the radiation is not important. Only one radiation source is necessary when the container and medical device have sufficient transmissivity to allow sufficient energy to transmit through the container and device to affect sterility on the entire contents of the container. For example, one radiation source may be effective for a container holding a contact lens which does not have an UV-blocker in its polymer, or a contact lens that is transmissive to greater than 30 %, more preferably greater than 50 % of UV radiation (240-280 nm).

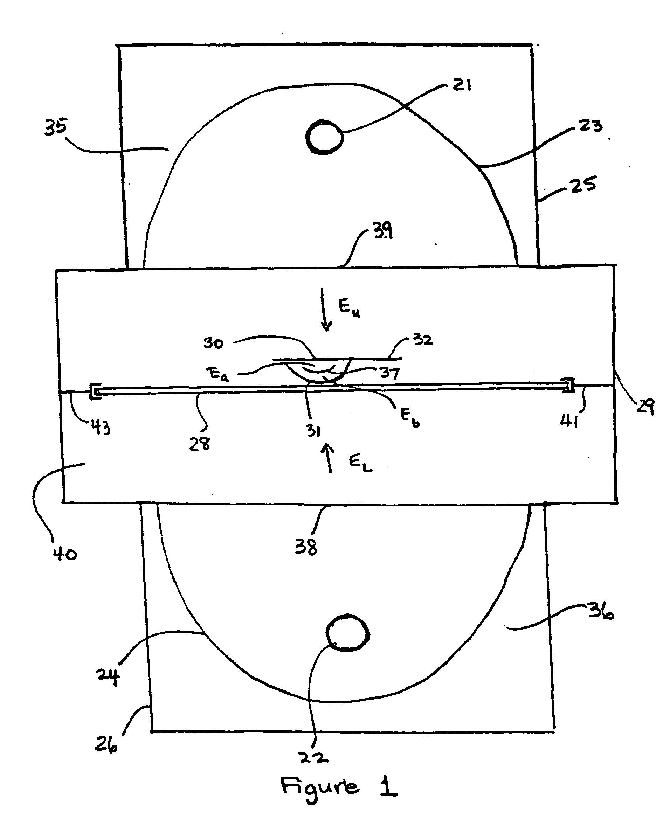

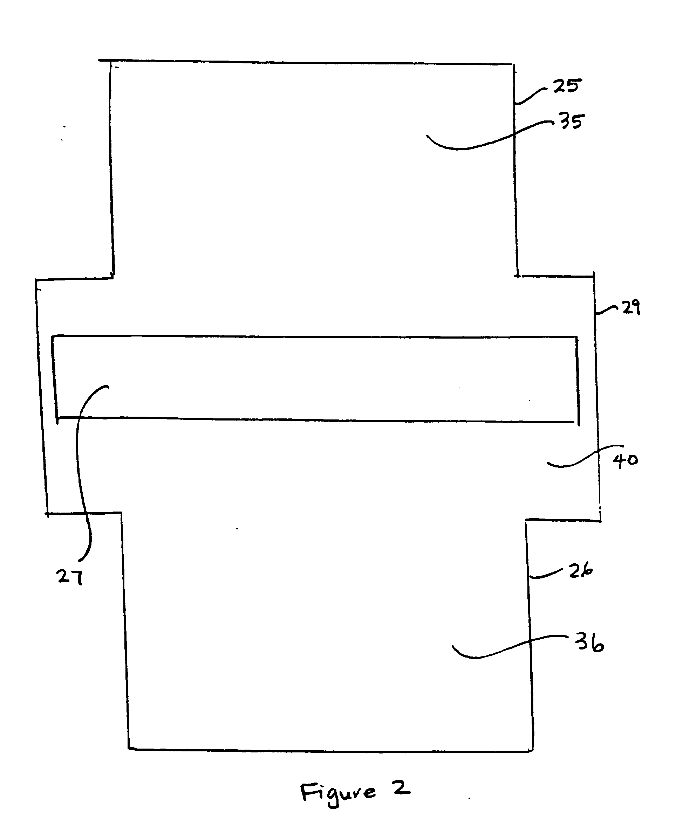

The presently preferred apparatus for delivering the high intensity radiation to the contact lens having a UV-blocker in a container with an aqueous solution in the container is a PurePulse PBS1-4 system (manufactured by PurePulse Technologies, Inc., San Diego, CA) which consists of two flash lamp assemblies connected in series, each of which consists of a lamp, and a reflector in a housing. The PBS1-4 system further consists of a pulse-generator capable of generating a large pulse of energy by virtue of its large capacitance (80-160 $\mu$F) and high potential (greater than 6 kV), and control circuits. Each lamp generates a broad spectrum of radiation which includes ultraviolet, infrared and visible light. The system is shown in Figures 1 and 2. Figure 1 shows two lamp assemblies 35 and 36. Lamp assembly 35 comprises lamp 21, reflector 23 and lamp housing 25, having a protective quartz plate 39. Lamp assembly 36 comprises lamp 22, reflector 24 and lamp housing 26, having a protective quartz plate 38. Also shown in Figure 1 is the chamber 40, which was added to the PBS1-4 system. The chamber comprises a drawer front 27, the product support 28, and spacer 29. The spacer 29 separates and supports the lamp assemblies and moveably holds the product support 28 via brackets 41 and 43 at a fixed distance from the lamp assemblies. The product support 28 is attached to the drawer front 27. Each flash lamp 21 and 22 contains a rare gas as a luminous component, preferably xenon, within a glass envelope or cylinder, preferably comprised of quartz, more preferably comprised of sapphire. The lamps comprising sapphire are preferred, because they can withstand higher internal pressures over a range of temperatures, produce less devitrification and have higher transmission coefficients over a broad range of wavelengths. The flash lamps are commercially available, for example from ILC Technology, Inc., Sunnyvale, CA. The lamp assemblies 35 and 36 face each other with a space between the lamps for the product support 28 holding the container or containers to be sterilized. The system is shown with one container 30 on the product support 28 ready for sterilization. The flash lamps flash substantially simultaneously, preferably within less than 5 microseconds, each generating a minimum of 40 mJ/cm$^2$ of UV radiation (240-280 nm), more preferably a minimum of 50 mJ/cm$^2$ (240-280 nm) per flash. The system is light-tight when the lamps flash, due to the chamber 40 which is rendered light-tight when the product support 28 is pushed into the space between the lamp assemblies 35 and 36 and the drawer front 27 creates a light-tight seal with the front of spacer 29. The system has at least one capacitor (not shown) which stores the energy at a high voltage needed to cause both lamps to flash substantially simultaneously. Each flash lamp 21 and 22 is at least partly surrounded by a reflector 23 and 24 which focuses light in the direction of the container 30. The container 30, comprises a contact lens 37 and aqueous solution (not shown). Preferably, the reflectors 23 and 24 have a cylindrical, polygonal, parabolic or elliptical shape and a semi-circular cross-section around the flash lamps. The reflectors are formed by extruding aluminum and adding reflective coatings. More information on flash lamp systems can be found in US Patents 4,464,336; 5,034,235; and 4,871,559.

The containers are preferably placed between the two lamps so that the contact lenses are closest to the center-line between the two lamps, and in the focal planes of the reflectors. Typically, multiple contact lens containers will be inserted between the lamps and flashed together to render the multiple containers of the contact lenses sterile simultaneously. If two rows of containers are to be flashed simultaneously, they are positioned in the center between the lamps facing bowl 31 to bowl with the tabs 32 facing outward. Preferably, 6 or more containers are flashed simultaneously, more preferably, 8 or more containers, most preferably 10 or more containers are flashed simultaneously. The presently preferred embodiment flashes 12 containers some of which can be removably-attached to each other. These removably-attached containers are commonly used to configure multi-packs of contact lens containers.

The contact lens containers can be inserted and removed from between the flash lamps by any means. Presently it is preferred that the lamps face each other with one facing in the direction of the floor and the other facing in the direction of the ceiling. Further, it is presently preferred that the lamps remain stationary and that a moveable product support 28 which behaves like a drawer is used to move the contact lens containers into and then out of the space between the lamps. The product support 28 which holds the one or more contact lens containers between the two lamps preferably comprises a quartz glass tray. Fused silica, synthetic fused silica, or sapphire may be substituted for the quartz glass. In operation, the tray is first loaded with the one or more containers to be flashed, the tray and attached drawer front are pushed into the support between the lamps which renders the chamber light-tight, the lamps are flashed, and after the containers have received the appropriate dose, delivered in at least one flash, the sterilized contact lens containers are removed from the tray. The containers are preferably placed on the product support with the bowl of the container closest to the flash lamp which faces the ceiling and the lid of the container closest to the flash lamp which faces the floor.

Alternatively, the product support can consist of any materials which will hold the containers and permit enough ultraviolet radiation (240-280 nm) to reach the contents of the one or more containers. For example, it can comprise polymeric or other glass materials in any configuration which holds the containers, e.g. sheet , mesh, or bars. It is presently preferred that the product support comprises quartz. Preferably, only when a light-tight chamber is established will the lamps be permitted to flash. A light-tight chamber will prevent any photo-annealing of the microorganisms' damaged DNA after subjecting the medical device to UV radiation (240-280 nm).

Alternatively, the lamps can be positioned facing in any direction. Preferably the lamps face each other. For example the lamps can be positioned facing opposite walls. The containers can either be stacked up within the chamber or more preferably an array of removably-attached containers can be hung between the lamps, for example by a hook, clasp, or clip, or the containers can be moved into the space between the lamps by a conveyor.

In the preferred embodiment, the distance between the lamp assemblies is determined by the focal planes of the reflectors. In the present embodiment, the surfaces of the container are 21 mm away from the lamp assemblies. Preferably the reflector is designed to minimize the working distance between the lamp assemblies, provided there is enough space between the lamp assemblies for insertion and removal of the containers. For the preferred embodiment, the reflector is preferably designed to focus at least 50 mJ/cm$^2$ UV radiation (240-280nm) and/or at least 3.5 J/cm$^2$ broad spectrum at the containers.

The energy levels specified herein can be used to determine the number of flashes which are needed to sterilize a medical device in a container. The transmissivity of a container and the medical device within the container must be determined. As taught herein, it is now known that the minimum level of energy which must reach the inside of the container and the surface of the medical device to obtain sterilization must be equivalent to at least 18 mJ/cm$^2$ of UV radiation in the range of 240-280 nm. Then, the following formula with reference to Figure 1 can be used to calculate the amount of energy. The following formula is suited for a system which has two energy sources, but can be modified if one or more than two energy sources are used:

Total energy from all sources $E(\lambda)=E_a(\lambda) + E_b(\lambda)$, where $E_a(\lambda)$ indicated in Figure 1 is the energy above the contact lens 37 in the container 30, and $_b(\lambda)$ indicated in Figure 1 is the energy below the contact lens 37 in the container 30. where

$$E_a(\lambda) = E_U(\lambda) \left[e^{-k(\lambda)}_{LS}{}^x_{LS}\right] + E_L(\lambda) \left[e^{-k(\lambda)}_{B}{}^x_{B}\right]\left[e^{-k(\lambda)}_{L}{}^x_{L}\right],$$

and

$$E_b(\lambda)= E_U(\lambda) \left[e^{-k(\lambda)}_{LS}{}^x_{LS}\right]\left[e^{-k(\lambda)}_{L}{}^x_{L}\right] + E_L(\lambda) \left[e^{-k(\lambda)}_{B}{}^x_{B}\right]$$

where transmissivity $T = I$ (intensity which penetrates a material)/$I_0$ (intensity incident on a material) = $e^{-k(\lambda)x}$ where $k(\lambda)$ is the transmissivity constant of a material and x is the thickness of the material. $E_U(\lambda)$ is the energy from the upper lamp indicated in Figure 1 by the arrow labeled $_U(\lambda)$. $E_L(\lambda)$ is the energy from the lower lamp indicated in Figure 1 by the arrow labeled $E_L(\lambda)$. The subscript LS indicates lidstock. The subscript L indicates lens. The subscript B indicates bowl. $E_a(\lambda)$ and $_b(\lambda)$ each have to be at least 18 mJ/cm$^2$ UV radiation (240-280 nm) to achieve sterilization of the medical device. If the transmissivity of the container or medical device is increased or decreased, the energy of the radiation sources can be calculated, because $E_a(\lambda)$ and $_b(\lambda)$ are known. (For the preferred embodiment herein, the contribution of the UV radiation (240-280 nm) which passes through the UV-blocking contact lens was assumed to be zero when determining the amount of UV radiation which the microorganism is subjected to on each side of the lens.)

Initially, a prototype system consisting of two lamps was evaluated capable of delivering 1.5 J/cm$^2$ broad spectrum from each lamp to the outside of the container. Since the ultraviolet radiation (240-280 nm) is the recognized vehicle of kill of the microorganism, it was necessary to quantify the output of these lamps for ultraviolet radiation (240-280 nm). Extensive lamp mapping studies showed the output of each lamp at 21 mm from the bottom of the lamp housing to be on average 25 mJ/cm$^2$ (240-280 nm) from each of the lamps to the containers for an area 60 mm wide by 180 mm long. A succession of microbiological tests on a battery of microorganisms were conducted to determine if sterility could be achieved.

Microbiological evaluation of the effectiveness of the system was conducted using containers, consisting of bowls and lidstock, (both about 50 percent transmissible to UV radiation (240-280 nm)), holding UV-blocking contact lenses (20% transmissive to 240-280 nm) in a non-preserved solution of buffered borate. The test microorganism was added at a concentration of $10^4$ colony forming unit/package (cfu/pkg). The closed intact containers were centered between the lamps and simultaneously subjected to 25 mJ/cm$^2$ (240-280 nm) from each lamp. One hundred containers containing <u>Aspergillus niger</u> (ATCC 16404), <u>Bacillus stearothermophilus</u> (ATCC 7953) spores or <u>Bacillus subtilis</u> (ATCC 9372) spores were exposed to 1-flash for a total of 50 mJ/cm$^2$ UV radiation (240-280 nm) (25 mJ/cmper lamp) from both the top and bottom lamps. The containers inoculated with <u>Aspergillus niger</u> (ATCC 16404) were then processed in a laminar flow hood whereby the entire contents of the container were placed into potato dextrose broth and incubated at 25° C for 14 days. The containers inoculated with <u>Bacillus</u> spore preparations were transferred to tubes containing 40 ml of trypticase soy broth and incubated for 14-days at 35-37°C. This tube terminal sterilization method allows for the detection of the viability of 1-single cell. After 14-days of incubation, the tubes were visually evaluated for turbidity and designated as positive for growth or negative for no growth. The positive tubes were subsequently identified and confirmed as the microorganism inoculated in the test. This experiment was repeated with 100 additional tubes for 2 flashes (50 mJ/cm$^2$ cumulative exposure from each lamp (240-280 nm)) both lamps flashing simultaneously, and repeated again for 3 flashes (75 mJ/cm$^2$ cumulative exposure from each lamp (240-280 nm)) both lamps flashing simultaneously. The number of test tubes with viable test microorganisms out of the one hundred tested at each energy level were recorded in Table 1.

The results in Table 1 clearly show that the amount of energy produced by the system was capable of inactivating some of the test microorganism; however, it was not effective in inactivating any of the more resistant spore formers. However, this system could be used to deliver enough energy in one flash to sterilize a non-UV-blocking contact lens in an aqueous solution in a container having transmissivity greater than 50% for 240-280 nm radiation.

TABLE 1 Number of positive tubes/100

| Microorganism | 1 flash * | 2 flashes * | 3 flashes * |
|---|---|---|---|
| <u>Aspergillus niger</u> (ATCC 16404) | 2 | 1 | 4 |
| <u>Bacillus stearothermophilus</u> (ATCC 7953) | 89 | 68 | 4 |
| <u>Bacillus subtilis</u> (ATCC 9372) | 100 | 81 | 29 |
| *each flash delivering 25 mJ/cm$^2$ (240-280 nm) per lamp | | | |

A series of modifications, including an increase in the capacitance of the power supply in the pulse generator, increased the full spectrum output of each lamp to a minimum of 3.5 J/cm$^2$ with the UV radiation output of 50 mJ/cm$^2$ (240-280 nm) over the same 60 mm x 180 mm treatment area. In this system the two lamps flashed simultaneously. A microbiological test of the output showed that increasing the UV-radiation output from 25 mJ/cm$^2$ to 50 mJ/cm$^2$ in the spectral region from 240 - 280 nm of each lamp resulted in sufficient UV radiation to sterilize <u>Aspergillus niger</u> (ATCC 16404), <u>Bacillus cereus</u> (an environmental isolate), <u>Bacillus subtilis</u> (ATCC 9372), <u>Candida albicans</u> (ATCC 10231), <u>Pseudomonas aeruginosa</u> (ATCC 9027), <u>Staphylococcus aureus</u> (ATCC 6538) and <u>Serratia marcescens</u> (ATCC 13880). The results of this test are listed in Table 2. Each cell in Table 2 indicates the number of containers out of 100 containers which tested positive, that is, which had viable test microorganisms in them.

TABLE 2

| Microorganism | 1 flash * | 2 flashes * | 3 flashes * |
|---|---|---|---|
| <u>Aspergillus niger</u> (ATCC 16404) | 0 | 0 | 0 |
| | 0 | 0 | 0 |
| | 0 | 0 | 0 |
| <u>Bacillus cereus</u> | 0 | 0 | 0 |
| <u>Bacillus stearothermophilus</u> (ATCC 7953) | 0 | 0 | 0 |
| | 0 | 0 | 0 |
| | 0 | 0 | 0 |
| <u>Bacillus subtilis</u> (ATCC 9372) | 1 | 0 | 0 |
| | 1 | 0 | 0 |
| | 1 | 0 | 0 |
| <u>Candida albicans</u> (ATCC 10231) | 0 | 0 | 0 |
| | 0 | 0 | 0 |
| | 0 | 0 | 0 |
| <u>Pseudomonas aeruginosa</u> (ATCC 9027) | 0 | 0 | 0 |
| | 0 | 0 | 0 |

Table continued

| Microorganism | 1 flash * | 2 flashes * | 3 flashes * |
|---|---|---|---|
| | 0 | 0 | 0 |
| Staphylococcus aureus (ATCC 6538) | 0 | 0 | 0 |
| | 0 | 0 | 0 |
| | 0 | 0 | 0 |
| Serratia marcescens (ATCC 13880) | 0 | 0 | 0 |
| | 0 | 0 | 0 |
| | 0 | 0 | 0 |
| * each flash delivering 50 mJ/cm$^2$ (240-280 nm) per lamp | | | |

While the modified machine proved capable of delivering the required amount of energy, the modifications reduced the lamp life from an expected 20-30 million flashes to 2-5 thousand. In manufacturing, it is not practical to consider running the machine with this reduced lamp life; therefore, the machine requires further modifications to extend the lamp life. We have determined that the reflectors need to be redesigned to focus the light energy from the existing lamps to the container to a smaller area of 25 mm x 180 mm, thus increasing the fluence of each reflector at the focal plane of the reflector to a minimum of 3.5 J/cm$^2$ of broad spectrum radiation and a minimum of 50 mJ/cm$^2$ (240-280 nm). For a 50 percent transmissive container, the reflector (and lamp) will deliver a minimum of 25 mJ/cm$^2$ UV radiation (240-280 nm) to the microorganisms in the container, because 50 % of the energy is absorbed or scattered by the container. Increasing the fluence from the reflectors focused onto the containers, will lower the required energy from the flash lamp, and result in an increase in the lamp life. Further, we determined that the capacitors will need to be upgraded to increase the size of the capacitor to between from 80 and 160 microFarads to produce more energy to increase the amount of UV radiation delivered to the container. The power supply will generate a potential of 2500-6000 volts. The energy will be discharged from the radiation source within 1 millisecond. The reflectors will have enhanced reflection in the ultraviolet. In a preferred embodiment, the reflectors will minimize the non-ultraviolet radiation reaching the medical device. The containers will be located in the focal plane of the reflectors where there is maximum flux.

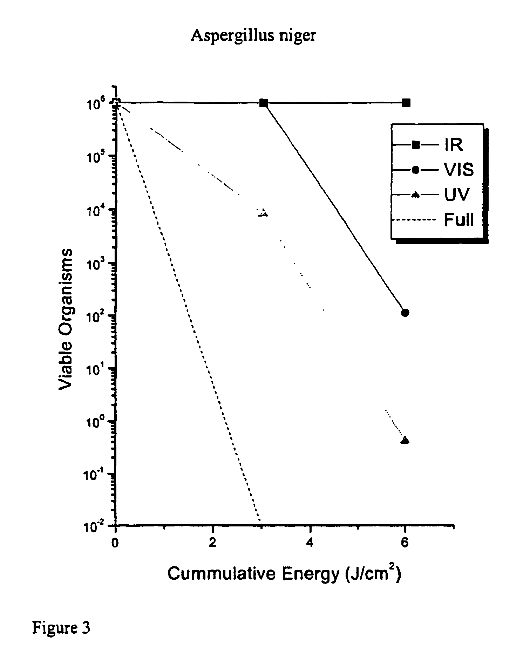

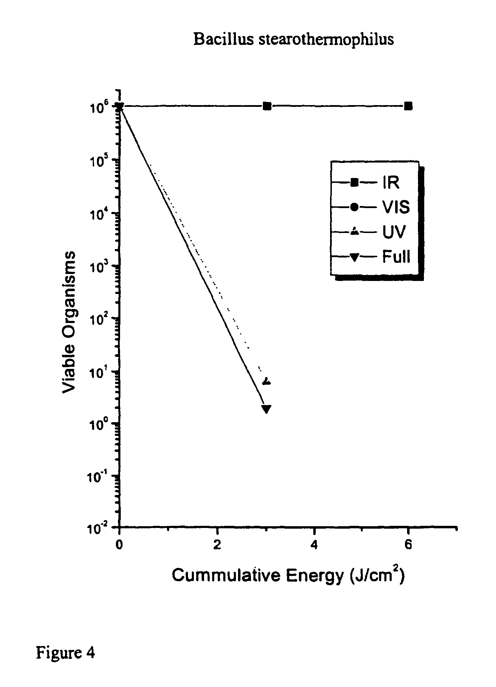

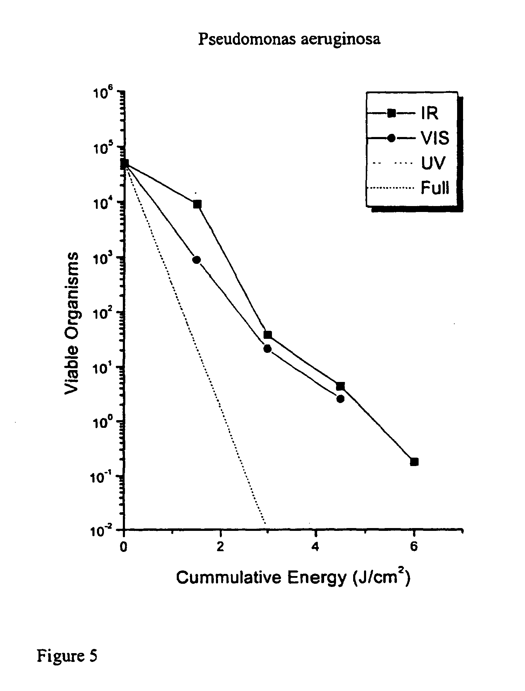

The criticality that at least a portion of the radiation delivered to the medical device be UV radiation was proven by exposing microorganisms to pulses of a broad spectrum of light (200-1100 nm), and as well as selected portions of the spectrum. Filters were utilized to separate the light spectrum (e.g. ultraviolet: 200 - 400 nm, visible: 400 - 700 nm, infrared: 700 - 1100 nm and full spectrum: 200 -1100 nm). In this experiment 10$^6$ cfu/container of Aspergillus niger (ATCC 16404) (Figure 3), Bacillus stearothermophilus (ATCC 7953) (Figure 4), and Pseudomonas aeruginosa (ATCC 9027) (Figure 5) were evaluated in a container having a 50% UV transmissive lidstock (240-280 nm) and bowl, and containing non-preserved solution. After inoculation, sample sets of 12 containers of each microorganism were flashed with two 3.5 J/cm$^2$ broad spectrum lamp systems with increasing amounts of energy and with a range of filters, and no filter. After the samples were flashed, the samples were diluted and inoculated into an agar medium in which to count the colony forming units. The results are depicted in Figures 3-5. Figure 3 shows the sensitivity of Aspergillus niger (ATCC 16404) to the different regions of the light spectrum. Figure 4 shows the sensitivity of Bacillus stearothermophilus (ATCC 7953) to the different regions of the light spectrum. In Figure 4, the lines for the IR and VIS overlap. Figure 5 shows the sensitivity of Pseudomonas aeruginosa (ATCC 9027) to the different regions of the light spectrum. In Figure 5, the lines for the UV and Full overlap The results show that the predominate spectral region for inactivation is the ultraviolet radiation (200-400 nm). Pseudomonas aeruginosa (ATCC 9027) was the microorganism most susceptible in all spectral regions.

An experiment was conducted to determine the sterilization efficacy as a function of radiation intensity, in which fractional positives were obtained at lower energy levels. In separate studies 10$^4$ cfu/pkg of Bacillus subtilis (ATCC 9372), Bacillus stearothermophilus (ATCC 7953), and Aspergillus niger (ATCC 16404) were added to a container having a 50 % UV-transmissive lidstock and bowl (240-280 nm) with UV-blocking contact lenses in non-preserved solutions. The inoculated containers were exposed to ultraviolet radiation (240 - 280 nm) from 10.2 to 48.6 mJ/cm$^2$/pulse per lamp for 1, 2 and 3 pulses. The system comprised two lamps flashing simultaneously. After exposure the entire contents of the container (contact lens and non-preserved solution) were added to growth medium and incubated at optimal growth temperatures for the test microorganisms. After 14 days incubation, the number of tubes for each set of twelve tubes having viable test microorganism was determined, and recorded in Table 3. Table 3 shows that the threshold of complete cell inactivation for Bacillus subtilis (ATCC 9372) is greater than 18.1 mJ/cm$^2$, for Bacillus stearothermophilus (ATCC 7953), it is greater than 30.7 mJ/cm$^2$, and for Aspergillus niger (ATCC 16404), it is greater than 18.1 mJ/cm$^2$.

TABLE 3

| Test Micro-Organism | No. of Flashes | UV radiation (240-280 nm) in mJ/cm² from each lamp | | | | | | | | | |
|---|---|---|---|---|---|---|---|---|---|---|---|
| | | 10.2 | 12.5 | 15.1 | 18.1 | 21.8 | 26.1 | 30.7 | 36.0 | 41.1 | 48.6 |
| B. sub. | 1 | 11 | 7 | 4 | 4 | 0 | 0 | 0 | 0 | 1 | 0 |
| | 2 | 0 | 0 | 0 | 0 | 0 | 0 | 0 | 0 | 0 | 0 |
| | 3 | 0 | 0 | 0 | 0 | 0 | 0 | 0 | 0 | 0 | 0 |
| B. stear. | 1 | 12 | 12 | 12 | 12 | 6 | 9 | 4 | 9 | 9 | 0 |
| | 2 | 12 | 12 | 12 | 10 | 0 | 0 | 0 | 0 | 0 | 0 |
| | 3 | 12 | 12 | 2 | 0 | 0 | 0 | 0 | 0 | 0 | 0 |
| Aspergillus niger (ATCC 16404) | 1 | 12 | 12 | 8 | 2 | 0 | 0 | 0 | 0 | 0 | 0 |
| | 2 | 8 | 2 | 1 | 0 | 0 | 0 | 0 | 0 | 0 | 0 |
| | 3 | 1 | 0 | 0 | 1 | 0 | 0 | 0 | 0 | 0 | 0 |

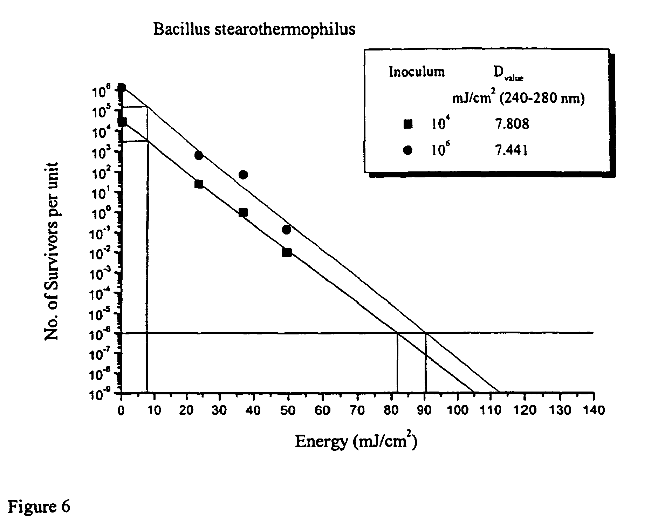

Figure 6 shows the graph of the average number of containers with Bacillus stearothermophilus (ATCC 7953) survivors in three sets of three containers tested at various UV radiation (240-280 nm) energy levels in mJ/cm² from 12 to 40 mJ/cm² for the fractional data. For the final exposure at 50 mJ/cm² (240-280 nm) three sets of one hundred containers were used, and the average number of positives is reported in the table. Due to the linearity of the data, the $D_{value}$ can be determined from the slope of the graph and by extrapolation a calculation can be made to determine a final dose which will render the package sterile at the desired sterility assurance level. As indicated on the graph the $D_{value}$ of Bacillus stearothermophilus (ATCC 7953) is 7.8 mJ/cm² to the container. The container had 50% transmissivity to UV radiation (240-280 nm); therefore, the $D_{value}$ is 3.9 mJ/cm² UV radiation (240-280nm) to the spores for an initial inoculum of $10^4$ spores. For an initial inoculum of $10^6$ spores, the $D_{value}$ of Bacillus stearothermophilus (ATCC 7953) is 7.4 mJ/cm² UV radiation (240-280 nm) to the container, or 3.7 mJ/cm² UV radiation (240-280nm) to the spores. This data was generated using the pair of 3.5 J/cm² broad spectrum lamps, pulsing simultaneously. The additional vertical lines on Figure 6 indicate the amount of UV radiation required to provide a sterility assurance level of $10^{-6}$ for an initial inoculum of $10^6$ and $10^4$ spores.

Although not preferred modes of this invention, it has been determined that this method of sterilization can be enhanced by adding chemicals, bactericides, surfactants, preservatives or heat to the contents of the container. Also the method of sterilization can be enhanced by shaking the container, or exposing the container to sonic vibration energy as it is exposed or between exposures to the radiation source or by heating the container.

This invention has been described with reference to particular embodiments; however, variations within the scope of the following claims are apparent to those of ordinary skill in the art.

**Claims**

1.   A process of sterilizing a contact lens that is hermetically sealed in a container against Bacillus stearothermophilus (ATCC 7953) to a sterility assurance level of at least $10^{-6}$ comprising: subjecting said contact lens in said container to at least 41 mJ/cm² ultraviolet radiation in the range of 240-280 nm to the spore.

2.   The process of claim 1 wherein to provide a sterility assurance level of $10^{-9}$ said spore is exposed to at least 52mJ/cm² of said UV radiation (240-280nm) during said subjecting step.

3.   The process of any one of claims 1 or 2, wherein said radiation is delivered to said spore by at least one pulsed radiation source.

4.   The process of claim 3, wherein said radiation is delivered by said pulsed radiation source in at most three pulses.

5.   The process of claim 3 or claim 4, wherein each pulse delivers at least 20 mJ/cm² UV radiation (240-280 nm) to said spore.

6.  The process of any one of claims 1 to 5, wherein at least 18 mJ/cm$^2$ UV radiation (240-280 nm) is delivered in less than 1 millisecond to said spore.

7.  The process of any one of claims 1 to 6, wherein said radiation is delivered by more than one radiation source.

8.  The process of claim 7, wherein said radiation sources are pulsed radiation sources and said radiation sources pulse substantially simultaneously.

9.  The process of claim 7 or claim 8, wherein said radiation sources are flash lamps.

10. The process of claim 9, wherein said flash lamps each comprise a reflector and a lamp wherein the fluence of each of said flash lamps at the focal plane of said reflector is at least 45 mJ/cm$^2$ UV radiation (240-280 nm).

11. The process of any one of claims 1 to 8, wherein said radiation is produced by laser.

12. The process of any one of claims 1 to 11, wherein said $D_{value}$ of <u>Bacillus stearothermophilus</u> (ATCC 7953) can be determined for said container by dividing 3.9 mJ/cm$^2$ by the transmissivity of said container exposed to said radiation source.

13. The process of claim 1 wherein the minimum total energy density of said ultraviolet radiation (240-280 nm) to microorganisms on said contact lens is at least 18 mJ/cm$^2$.

14. The process of claim 13, wherein the minimum total energy density of said ultraviolet radiation (240-280 nm) to said microorganisms is at least 30 mJ/cm$^2$, preferably at least 36 mJ/cm$^2$.

15. The process of claim 13 or claim 14, wherein said minimum total energy density of said ultraviolet radiation (240-280 nm) is delivered to said microorganisms in less than 20 seconds, preferably in less than 1 second, more preferably in less than 1 millisecond.

16. The process of any one of claims 13 to 15, wherein said radiation is provided by a pulsed radiation source which provides at least 20 mJ/cm$^2$ ultraviolet radiation (240-280 nm) per pulse to said microorganisms.

17. The process of any one of claims 15 to 16, wherein minimum total energy density of said ultraviolet radiation (240-280 nm) which reaches said microorganisms, further reaches said contents of said container whereby the entire contents of said container and said medical device are sterilized.

18. The process of claim 17, wherein said sealed container further comprises a non-preserved aqueous solution.

19. The process of claim 18, wherein said container is transmissive to at least 50% of said ultraviolet radiation (240-280 nm).

20. The process of claim 19, wherein said container is transmissive to at least 50% of said radiation (240-280 nm) in substantially all directions.

21. The process of claim 20, wherein said container comprises a lid and a bowl, wherein said lid and said bowl comprise thermoplastics and said lid and said bowl are transmissive to at least 50% of said radiation (240-280 nm) in substantially all directions.

22. The process of any one of claims 1 to 21, wherein prior to said subjecting step is the step of modifying radiation from a radiation source to eliminate wavelengths which would damage said contact lens.

23. The process of claim 13, wherein said contact lens blocks at least 50% of the UV radiation (240-280 nm).

24. The process of claim 13 or claim 23, wherein said subjecting step follows the steps of:

> (a) forming a contact lens;
> (b) placing said contact lens in a container; and
> (c) moving said container into an apparatus comprising a radiation source; and wherein said apparatus is light-tight during said subjecting step.

25. The process of claim 24, wherein the amount of radiation in the range of 240-280 nm delivered to the surfaces of said contact lens is between 18 mJ/cm$^2$ and 35 mJ/cm$^2$.

26. The process of claim 25, wherein said contact lens comprises a UV-blocker which blocks greater than 50% of the radiation between 240-280 nm.

27. The process of claim 26, wherein the amount of said ultraviolet radiation (240-280 nm) delivered to said contact lens is between 18 mJ/cm$^2$ and 150 mJ/cm$^2$.

28. The process of claim 27, wherein said flash lamps deliver at least 80 mJ/cm$^2$, preferably at least 100 mJ/cm$^2$, total UV radiation (240-280 nm) per flash to said container.

**Patentansprüche**

1. Verfahren zum Sterilisieren einer Kontaktlinse, die in einem Behälter hermetisch verschlossen ist, gegen *Bacillus stearothermophilus* (ATCC 7953) auf einen gesicherten Sterilisationsgrad von wenigstens 10$^{-6}$ umfassend: Unterziehen der Kontaktlinse in dem Behälter von mindestens 41 mJ/cm$^2$ an ultravioletter Bestrahlung im Bereich von 240 - 280 nm auf die Spore.

2. Verfahren nach Anspruch 1, wobei zur Bereitstellung eines gesicherten Sterilisationsgrads von 10$^{-9}$ die Spore mindestens 52 mJ/cm$^2$ der UV-Strahlung (240 - 280 nm) während dem Unterziehungsschritt ausgesetzt wird.

3. Verfahren nach einem der Ansprüche 1 bis 2, wobei die Strahlung durch wenigstens eine gepulste Strahlungsquelle auf die Spore abgegeben wird.

4. Verfahren nach Anspruch 3, wobei die Strahlung durch die gepulste Strahlungsquelle in maximal drei Pulsen zugeführt wird.

5. Verfahren nach Anspruch 3 oder Anspruch 4, wobei jeder Puls wenigstens eine 20 mJ/cm$^2$ UV-Strahlung (240 - 280 nm) zu der Spore zuführt.

6. Verfahren nach einem der Ansprüche 1 bis 5, wobei mindestens 18 mJ/cm$^2$ UV-Strahlung (240 - 280 nm) in weniger als 1 Millisekunde auf die Spore abgegeben werden.

7. Verfahren nach einem der Ansprüche 1 bis 6, wobei die Strahlung durch mehr als eine Strahlungsquelle abgegeben wird.

8. Verfahren nach Anspruch 7, wobei die Strahlungsquellen gepulste Strahlungsquellen sind und die Strahlungsquellen im wesentlichen simultan pulsieren.

9. Verfahren nach Anspruch 7 oder Anspruch 8, wobei die Strahlungsquellen Blitzlampen sind.

10. Verfahren nach Anspruch 9, wobei die Blitzlampen jeweils einen Reflektor und eine Lampe umfassen, wobei die Fluenz jeder der Blitzlampen in der Brennebene des Reflektors mindestens 45 mJ/cm$^2$ UV-Strahlung (240 - 280 nm) ist.

11. Verfahren nach einem der Ansprüche 1 bis 8, wobei die Strahlung durch einen Laser erzeugt wird.

12. Verfahren nach einem der Ansprüche 1 bis 11, wobei der D$_{Wert}$ von *Bacillus stearothermophilus* (ATCC 7953) für den Behälter durch Division von 3,9 mJ/cm$^2$ durch die Durchlässigkeit des Behälters, wenn der Strahlungsquelle ausgesetzt wird, bestimmt werden kann.

13. Verfahren nach Anspruch 1, wobei die minimale Gesamtenergiedichte der ultravioletten Strahlung (240 - 280 nm) auf die Mikroorganismen auf der Kontaktlinse mindestens 18 mJ/cm$^2$ ist.

**14.** Verfahren nach Anspruch 13, wobei die minimale Gesamtenergiedichte der ultravioletten Strahlung (240 - 280 nm) auf die Mikroorganismen wenigstens 30 mJ/cm$^2$, bevorzugt mindestens 36 mJ/cm$^2$ ist.

**15.** Verfahren nach Anspruch 13 oder Anspruch 14, wobei die minimale Gesamtenergiedichte der ultravioletten Strahlung (240 - 280 nm) in weniger als 20 Sekunden, bevorzugt in weniger als 1 Sekunde, weiter bevorzugt in weniger als 1 Millisekunde, den Mikroorganismen zugeführt wird.

**16.** Verfahren nach einem der Ansprüche 13 bis 15, wobei die Strahlung durch eine gepulste Strahlungsquelle bereitgestellt wird, die mindestens 20 mJ/cm$^2$ ultraviolette Strahlung (240 - 280 nm) pro Puls auf die Mikroorganismen bereitstellt.

**17.** Verfahren nach einem der Ansprüche 15 bis 16, wobei die minimale Gesamtenergiedichte der ultravioletten Strahlung (240 - 280 nm), die die Mikroorganismen erreicht, weiter die Inhalte des Behälters erreicht, wodurch die gesamten Inhalte des Behälters und der medizinischen Vorrichtung sterilisiert werden.

**18.** Verfahren nach Anspruch 17, wobei der verschlossene Behälter weiterhin eine nicht konservierte wäßrige Lösung umfaßt.

**19.** Verfahren nach Anspruch 18, wobei der Behälter für mindestens 50 % der Ultraviolettstrahlung (240 - 280 nm) durchlässig ist.

**20.** Verfahren nach Anspruch 19, wobei der Behälter für mindestens 50 % der Strahlung (240 - 280 nm) im wesentlichen in alle Richtungen durchlässig ist.

**21.** Verfahren nach Anspruch 20, wobei der Behälter einen Deckel und eine Schale umfaßt, wobei der Deckel und die Schale Thermoplasten umfassen, und der Behälter und die Schale für mindestens 50 % der Strahlung (240 - 280 nm) im wesentlichen in alle Richtungen durchlässig sind.

**22.** Verfahren nach einem der Ansprüche 1 bis 21, wobei vor dem Unterziehungsschritt ein Schritt zur Modifizierung der Strahlung der Strahlungsquelle ist, um Wellenlängen zu eliminieren, die die Kontaktlinse beschädigen würden.

**23.** Verfahren nach Anspruch 13, wobei die Kontaktlinse mindestens 50 % der UV-Strahlung (240 - 280 nm) blockiert.

**24.** Verfahren nach Anspruch 13 oder Anspruch 23, wobei der Unterziehungsschritt den Schritten folgt von

(a) Formen einer Kontaktlinse;
(b) Plazieren der Kontaktlinse in einen Behälter; und
(c) Bewegen des Behälters in eine Apparatur, die eine Strahlungsquelle umfaßt; und wobei die Apparatur während des Unterziehungsschrittes lichtdicht ist.

**25.** Verfahren nach Anspruch 24, wobei die Menge an Strahlung im Bereich von 240 - 280 nm, die auf die Oberflächen der Kontaktlinse abgegeben wird, zwischen 18 mJ/cm$^2$ und 35 mJ/cm$^2$ liegt.

**26.** Verfahren nach Anspruch 25, wobei die Kontaktlinse einen UV-Blocker umfaßt, der mehr als 50 % der Strahlung zwischen 240 - 280 nm blockiert.

**27.** Verfahren nach Anspruch 26, wobei die Menge der ultravioletten Strahlung (240 - 280 nm) die auf die Kontaktlinse abgegeben wird, zwischen 18 mJ/cm$^2$ und 150 mJ/cm$^2$ liegt.

**28.** Verfahren nach Anspruch 27, wobei die Blitzlampen mindestens 80 mJ/cm$^2$, bevorzugt mindestens 100 mJ/cm$^2$ an Gesamt-UV-Strahlung (240 - 280 nm) pro Blitz auf den Behälter abgeben.

## Revendications

**1.** Procédé de stérilisation d'une lentille de contact qui est hermétiquement scellée dans un conteneur contre *Bacillus stearothermophilus* (ATCC 7953) à un degré de garantie de stérilité d'au moins 10$^{-6}$ comprenant l'étape consistant à soumettre ladite lentille de contact dans ledit conteneur à un rayonnement ultraviolet d'au moins 41 mJ/cm$^2$ dans

la gamme de 240 à 280 nm dirigé vers le spore.

**2.** Procédé selon la revendication 1 dans lequel ledit spore est exposé à au moins 52 mJ/cm$^2$ dudit rayonnement ultraviolet (240 à 280 nm) durant ladite étape de soumission de la lentille, de façon à obtenir un degré de garantie de stérilité d'au moins 10$^{-9}$.

**3.** Procédé selon la revendication 1 ou 2, dans lequel ledit rayonnement est envoyé vers ledit spore par au moins une source de rayonnement par impulsion.

**4.** Procédé selon la revendication 3, dans lequel ledit rayonnement est administré par ladite source de rayonnement par impulsion en trois impulsions au plus.

**5.** Procédé selon la revendication 3 ou 4, dans lequel chaque impulsion envoie au moins 20 mJ/cm$^2$ de rayonnement ultraviolet (240 à 280 nm) vers ledit spore.

**6.** Procédé selon l'une quelconque des revendications 1 à 5, dans lequel au moins 18 mJ/cm$^2$ de rayonnement ultraviolet (240 à 280 nm) sont envoyés en moins d'1 milliseconde vers ledit spore.

**7.** Procédé selon l'une quelconque des revendications 1 à 6, dans lequel ledit rayonnement est administré par plusieurs sources de rayonnement.

**8.** Procédé selon la revendication 7, dans lequel lesdites sources de rayonnement sont des sources de rayonnement par impulsion et lesdites sources de rayonnement fournissent une impulsion sensiblement simultanée.

**9.** Procédé selon la revendication 7 ou 8,
dans lequel lesdites sources de rayonnement sont des lampes flash.

**10.** Procédé selon la revendication 9, dans lequel lesdites lampes flash comprennent chacune un réflecteur et une lampe où la fluence de chacune desdites lampes flash au niveau du plan focal dudit réflecteur est au moins de 45mJ/cm$^2$ de rayonnement ultraviolet (240 à 280 nm).

**11.** Procédé selon l'une quelconque des revendications 1 à 8, dans lequel ledit rayonnement est produit par un laser.

**12.** Procédé selon l'une quelconque des revendications 1 à 11, dans lequel ladite D$_{valeur}$ de *Bacillus stearothermophilus* (ATCC 7953) peut être déterminée pour ledit conteneur en divisant 3,9 mJ/cm$^2$ par la transmissivité dudit conteneur exposé à ladite source de rayonnement.

**13.** Procédé selon la revendication 1, dans lequel la densité minimale énergétique totale dudit rayonnement ultraviolet (240 à 280 nm) envoyée vers les microorganismes sur ladite lentille de contact est d'au moins 18 mJ/cm$^2$.

**14.** Procédé selon la revendication 13, dans lequel la densité minimale énergétique totale dudit rayonnement ultraviolet (240 à 280 nm) envoyée vers lesdits microorganismes est d'au moins 30 mJ/cm$^2$, de préférence d'au moins 36 mJ/cm$^2$.

**15.** Procédé selon la revendication 13 ou 14, dans lequel ladite densité minimale énergétique totale dudit rayonnement ultraviolet (240 à 280 nm) est envoyée vers lesdits microorganismes en moins de 20 secondes, de préférence en moins de 1 seconde, de préférence en moins de 1 milliseconde.

**16.** Procédé selon l'une quelconque des revendications 13 à 15, dans lequel ledit rayonnement est administré par une source de rayonnement par impulsion qui envoie au moins 20 mJ/cm$^2$ de rayonnement ultraviolet (240 à 280 nm) par impulsion vers lesdits microorganismes.

**17.** Procédé selon l'une quelconque des revendications 15 ou 16, dans lequel la densité minimale énergétique totale dudit rayonnement ultraviolet (240 à 280 nm) qui atteint lesdits microorganismes atteint en outre ledit contenu dudit conteneur moyennant quoi le contenu entier dudit conteneur et ledit dispositif médical sont stérilisés.

**18.** Procédé selon la revendication 17, dans lequel ledit conteneur scellé comprend en outre une solution aqueuse sans agent de conservation.

**19.** Procédé selon la revendication 18, dans lequel ledit conteneur laisse passer au moins 50 % dudit rayonnement ultraviolet (240 à 280 nm).

**20.** Procédé selon la revendication 19, dans lequel ledit conteneur laisse passer au moins 50 % dudit rayonnement ultraviolet (240 à 280 nm) dans sensiblement toutes les directions.

**21.** Procédé selon la revendication 20, dans lequel ledit conteneur comprend un opercule et une cupule, dans lequel ledit opercule et ladite cupule comprennent des thermoplastiques et ledit opercule et ladite cupule laissent passer au moins 50 % dudit rayonnement (240 à 280 nm) dans sensiblement toutes les directions.

**22.** Procédé selon l'une quelconque des revendications 1 à 21, dans lequel avant ladite étape de soumission de la lentille a lieu une étape de modification du rayonnement à partir d'une source de rayonnement pour éliminer les longueurs d'onde qui altéreraient ladite lentille de contact.

**23.** Procédé selon la revendications 13, dans lequel ladite lentille de contact bloque au moins 50 % du rayonnement ultraviolet (240 à 280 nm).

**24.** Procédé selon la revendications 13 ou 23, dans lequel ladite étape de soumission de la lentille suit les étapes consistant à:

(a) former une lentille de contact;
(b) placer ladite lentille de contact dans un conteneur ; et
(c) déplacer ledit conteneur dans un appareil comprenant une source de rayonnement ; et dans lequel ledit appareil est imperméable à la lumière durant ladite étape de soumission de la lentille.

**25.** Procédé selon la revendications 24, dans lequel la quantité de rayonnement dans la gamme de 240 à 280 nm envoyée vers les surfaces de ladite lentille de contact est comprise entre 18 mJ/cm$^2$ et 35 mJ/cm$^2$.

**26.** Procédé selon la revendication 25, dans lequel ladite lentille comprend un anti-UV qui bloque plus de 50 % des rayonnements entre 240 et 280 nm.

**27.** Procédé selon la revendication 26, dans lequel la quantité dudit rayonnement ultraviolet (240 à 280 nm) envoyée vers ladite lentille de contact est comprise entre 18 mJ/cm$^2$ et 150 mJ/cm$^2$.

**28.** Procédé selon la revendication 27, dans lequel lesdites lampes flash envoient au moins 80 mJ/cm$^2$, de préférence au moins 100 mJ/cm$^2$ de rayonnement ultraviolet total (240 à 280 nm) par flash vers ledit conteneur.

Figure 1

Figure 2

Aspergillus niger

Figure 3

Bacillus stearothermophilus

Figure 4

Pseudomonas aeruginosa

Figure 5

Bacillus stearothermophilus

Figure 6